# EUROPEAN PATENT SPECIFICATION

(11) **EP 4 007 921 B1**
(45) Date of publication and mention of the grant of the patent: **25.03.2026**
(21) Application number: 20753452.0
(22) Date of filing: 31.07.2020
(51) Int. Cl.: G01N 33/68

(54) **METHOD OF DETERMINING RISK FOR CHRONIC STRESS AND STROKE**
VERFAHREN ZUR BESTIMMUNG DES RISIKOS VON CHRONISCHEM STRESS UND SCHLAGANFALL
MÉTHODE DE DÉTERMINATION DE RISQUE DE STRESS CHRONIQUE ET D'ACCIDENT VASCULAIRE CÉRÉBRAL

(30) Priority: 01.08.2019 ZA 201905103
(43) Date of publication of application: 08.06.2022
(73) Proprietor: North-West University, 2531 Potchefstroom (ZA)
(72) Inventor: MALAN, Leoné, 2531 Potchefstroom (ZA); MALAN, Nicolaas, Theodor, 2531 Potchefstroom (ZA)
(74) Representative: Michalski Hüttermann & Partner Patentanwälte mbB
(86) International application number: PCT/IB2020/057269
(87) International publication number: WO 2021/019511

(56) References cited:
- EP-A1- 2 927 692
- MEHRSHAD VAFAIE ET AL: "High-sensitivity cardiac troponin T as an independent predictor of stroke in patients admitted to an emergency department with atrial fibrillation", PLOS ONE, vol. 14, no. 2, 12 January 2019 (2019-01-12), pages e0212278, XP055735998, DOI: 10.1371/journal.pone.0212278
- WENTZEL ANNEMARIE ET AL: "Retinal Vasculature Reactivity During Flicker Light Provocation, Cardiac Stress and Stroke Risk in Africans: The SABPA Study", TRANSLATIONAL STROKE RESEARCH, SPRINGER US, BOSTON, vol. 10, no. 5, 9 November 2018 (2018-11-09), pages 485 - 494, XP036883198, ISSN: 1868-4483, [retrieved on 20181109], DOI: 10.1007/S12975-018-0673-4

## Description

### TECHNICAL FIELD

The present invention relates to methods of determining a STRESS^{risk} index for the risk of developing chronic stress and ischemic heart disease related stroke. Additionally, the invention relates to a method for determining a STRESS^{d-RISK} index for the risk of developing chronic stress and diabetes related stroke. More specifically, the disclosure relates to an early prognostic index that can be used to predict chronic stress and stroke risk.

### BACKGROUND TO THE INVENTION

Despite significant advances in medical technology and treatment programs, cardiovascular disease (CVD) and stroke remains the leading cause of death for both men and women worldwide. The American Heart Association lists seven key health and behavioral factors that increase risk for heart disease and stroke. This list does not include chronic emotional stress (hereafter stress), despite the fact that the World Health Organization (WHO) regards stress as one of the leading causes of disability worldwide. An individual that experiences increased levels of stress could therefore also experience an increased risk of developing stroke.

The effective management of stress could be beneficial to the prevention and therapy of ischemic heart disease and stroke and could be of major public health importance. Furthermore, individuals can be screened for elevated levels of several risk factors that could contribute to chronic stress and stroke. For example, elevated low-density-lipoprotein cholesterol (LDL) levels have been shown to increase the risk of developing stroke. The protein Troponin T (Trop T) may also be indicative of chronic stress, where increased levels of Trop T can be associated with tissue damage in heart muscle and may support the differential diagnosis of coronary versus non-coronary heart diseases.

Whilst stress has often been excluded as a risk factor for ischemic heart disease and stroke, accumulating data would suggest that stress may trigger perfusion deficits leading to ischemic heart disease and stroke risk. The overlap between symptoms of perfusion deficits and stress, such as palpitations, chest pain, and shortness of breath regularly occurring in healthy persons at emergency departments, makes it difficult to utilize mental health status as a diagnostic tool in ischemic heart disease. However, perfusion deficit symptoms are regularly treated and risk factors are evaluated without addressing emotions as a possible contributing factor in the condition. Moreover, the hesitancy of patients to discuss mental health issues may also increase the risk of stroke.

Presently, screening tests and preventative measures for chronic stress and stroke are limited. An index or tool that utilizes statistical analyses of various risk factors or markers of each individual might therefore prove to be useful as a predictor of chronic stress and stroke risk. The index or tool might further provide an early prognostic index in healthy or high-risk individuals in preventive medicine via phenotyping. Phenotyping explains how genetic and environmental influences come together to create an organism's physical appearance and behavior. Such a phenotype can be determined by measuring risk factors that can be analysed from a sample that has been obtained from an individual. Other risk factors may include, but are not limited to, age, gender, systolic blood pressure, hypertensive drugs, diabetes and smoking habit. The various risk factors can then be individually weighted and contribute to a final index or tool, which can be capable of determining or predicting whether an individual has a high risk of developing chronic stress and stroke.

### OBJECT OF THE INVENTION

Accordingly, it is an object of the present invention to provide a stress screening tool and with which the applicant believes the aforementioned disadvantages may at least partially be alleviated or which may provide a useful alternative for the known systems and methods.

### SUMMARY OF THE INVENTION

The invention is defined by the features of the independent claims.

According to a first aspect of the invention, there is provided a method for determining a STRESS^{risk} index for the risk of developing chronic stress and ischemic heart disease related stroke including:
i) Performing a multiple stepwise linear regression of biomarkers and risk factors, both transformed to be normally distributed, of an adapted UCLA score, wherein said UCLA score is a 10-year stroke risk composite score of the University of California, Los Angeles (UCLA) and, which includes the variables: individual's medical history; i.e., cardiovascular disease, kidney disease, myocardial infarction, diabetes and hypertension medication usage; demographic and lifestyle factors; i.e., age, race, sex, diabetes, smoking, alcohol use and physical activity habits; systolic and diastolic blood pressure; fibrinogen; waist circumference; perfusion deficits, i.e., myocardial ischemia; electrocardiography atrial fibrillation; and electrocardiography left ventricular hypertrophy;
ii) wherein the biomarkers and risk factors have been determined by obtaining a fasting biological sample i.e., blood, urine, and saliva from individuals; measuring the levels of the following biomarkers in the biological samples obtained from the individuals and measuring the levels of the following clinical markers of the individuals; wherein the biomarkers are serum cotinine values derived from a homogeneous immunoassay, gamma-glutamyl transferase (γ-GT), lipids and high sensitivity c-reactive protein (CRP) analyzed with an enzyme rated method, whole blood EDTA glycated hemoglobin (HbA1C) analyzed with turbidimetric inhibition immunoassay, citrate fibrinogen values derived by viscosity-based clotting method Immuno-turbimetric method 540 nm, saliva cortisol analyzed by an electrochemiluminescence immunoassay kit, serum cortisol determined with an electrochemiluminescence immunoassay, adrenocorticotrophic hormone (ACTH) determined with an electrochemiluminescence immunoassay, high sensitivity cardiac troponin determined with an electrochemiluminescence immunoassay, wherein acidified urine was collected overnight for urinary norepinephrine and epinephrine analysis using a 3-Cat Urine ELISA FAST TRACK kit, and wherein urine creatinine was measured using the enzymatic method; wherein the clinical markers are systolic blood pressure (SBP), diastolic blood pressure (DBP) both measured with a combined ambulatory blood pressure-electrocardiogram apparatus, silent myocardial ischemia (SMI) events or perfusion deficits assessed by two channel 24h electrocardiogram recordings, electrocardiography (ECG), atrial fibrillation identified by a resting 12-lead ECG, ECG left ventricular hypertrophy (ECG-LVH) determined by a 12-lead ECG, retinal vessel calibers measured as monochrome images using a retinal vessel analyzer and vessel-map-software, intra-ocular pressure (IOP) measured with a Applanation Tonometer, and diastolic ocular perfusion pressure (DOPP) calculated from DBP minus IOP;
iii) Performing a Receiver Operating Characteristic (ROC) analysis to assess the difference between the distribution of biomarkers and the distribution of risk factors at all classification thresholds;
iv) Determining the STRESS^{risk} index for three or more possible combinations of continuous biomarkers as an area under the curve (AUC) as a maximum of the ROC analysis, when discriminating the positive and negatives of the UCLA composite dichotomous biomarker denoted as Y, wherein the STRESS^{risk} index AUC was 0.77 (95% CI 0.27, 0.82) for a positive prediction (85% sensitivity, 48% specificity);
v) Determining a cut-off value by the Youden index for the STRESS^{risk} index that maximizes correct classifications and/or minimizes incorrect classifications and denotes the combination of biomarkers at the determined optimal cut-off value as biomarker V;
vi) Validating biomarker V by using Y as the dependent variable and V as the predicted probability of positives using a logistic regression on the selected input continuous biomarkers and confounding risk factors;
vii) Discriminating the AUC between the positives and negatives of Y using the predicted probability of positives and using the sensitivity and specificity of correct predictions as diagnostic for predictive validity;
viii) Determining an optimal cut-off value for V using ROC analysis;
ix) Using a non-linear regression model that includes neural networks as comparison to the logistic regression model;
x) Determining the maximum of the Youden index using the ROC curves with the non-linear regression analysis substantiating the functional relationship between the models using multilayer perceptron with two layers and trained with Bayesian regularization, wherein hidden layers have tansig functions and the output layer is linear with ten bootstrap repetitions;
xi) Optimizing the network and extracting the functional relationship with the UCLA stroke risk scores.

According to a second aspect of the invention, there is provided a method for determining a STRESS^{d-RISK} index for the risk of developing chronic stress and diabetes related stroke including:
i) Using the STRESS^{risk} index determined as described above to establish the STRESS^{d-RISK} index;
ii) Performing a statistical analysis of biomarkers and risk factors, wherein variables with skewed non-normal distributions were logarithmically transformed; wherein the biomarkers and risk factors are the biomarkers and risk factors of the adapted UCLA score, with the following nine independent markers: age, sex, systolic blood pressure, self-reported use of hypertensive drugs, diabetes, smoking habit, perfusion deficits, ECG atrial fibrillation and ECG-LVH, wherein the self-reported variables were replaced with quantitative markers, i.e. HbA1C ≥ 6.5% as a marker for diabetes, and nicotine metabolite cotinine ≥ 14 ng/ml as a marker for smoking, wherein in addition the liver enzyme gamma glutamyl transferase (GGT) was used as a marker for alcohol abuse; wherein the quantitative markers were determined by measuring in fasting blood samples: serum cotinine values determined by a homogeneous immunoassay, gamma glutamyl transferase (GGT) measured in serum, lipids and high sensitivity c-reactive protein measured in whole blood EDTA samples with an enzymatic colorimetric assay, total insulin-like growth factor-1 determined in serum using an immunoradiometric assay, and whole blood EDTA glycated hemoglobin (HbA1C) analyzed in with turbidimetric inhibition immunoassays;
iii) Determining standardized values of the adapted UCLA score by principal component analysis at baseline;
iv) Computing the first principal component scores as a weighted mean of standardized variables with determined weights reflecting the following seven component loadings: cotinine, GGT, diabetes defined as HbA1C ≥ 6.5%, systolic blood pressure, perfusion deficits, atrial fibrillation and ECG-LVH;
v) Determining the STRESS^{d-RISK} index by multiplying the component scores values by ten and increasing it by fifty, such that the mean of the STRESS^{d-RISK} index is 50 and its standard deviation lies between 0 and minus 100;
vi) Determining a cut-point for the STRESS^{d-RISK} index by conducting a ROC analysis using the cut-off determined in claim 1 iv), to discriminate between the positive and negative data and the sensitivity, specificity and percentage of correct predictions;
vii) Denoting the dichotomous variable, which discriminates between those respondents above the cut point and those below the cut point, as Y, wherein the STRESS^{d-RISK} index AUC was 0.78 (95% CI 0.73, 0.83) for a positive prediction (81% sensitivity, 59% specificity);
viii) Validating the STRESS^{d-RISK} index by applying multivariate linear regression analysis in a model using logarithmic transformed predictors in a complete dataset of N=349, by using subsets of 10 training sets with each 60% of population and 10 test sets with the remaining 40% of population;
ix) Applying logistic linear regression in a model by using logarithmic transformed predictors in all data as in viii) using variable Y as in vii);
x) Validating the logistic linear regression model by repeating model fitting on 10 randomly selected samples and 10 test sets;
xi) Predicting the probability of risk for chronic stress and diabetes related stroke by obtaining the maximum likelihood estimates of regression coefficients of all regressions;
xii) Using the dichotomous marker Y of the STRESS^{d-RISK} index as in vii) to discriminate between the positive and negatives of the markers of xi);
xiii) Using a logistic regression analysis wherein Y is used as the dependent variable and V contains the selected input continuous stress biomarkers as predictors of positives, wherein the STRESS^{d}-^{risk} index AUC was 0.82 (95% CI 0.75, 0.85) for a positive prediction (85% sensitivity, 58% specificity);
xiv) Determining an optimal cut-off value for V using ROC analysis;
xv) Using the area under the curve (AUC) in the ROC analysis on V, using Y, and the sensitivity, specificity and percentage correct predictions at the cut-off value as diagnostics for the predictive validity of V; and
xvi) Performing a Hosmer-Lemeshow test for testing the goodness of fit for the logistic regression risk prediction models in all participants, training and test sets.

In an embodiment of the invention, the individual may be a human.

The biological sample obtained from the individual may be selected from the group consisting of blood, serum, plasma, urine, saliva and a combination of one or more thereof.

In an embodiment of the invention, the one or more biomarkers may be selected from the group consisting of adrenocorticotrophic hormone (ACTH), cortisol, catecholamines (norepinephrine, epinephrine), low-density lipoprotein (LDL), high-density lipoprotein (HDL), troponin T (Trop T), gamma-glutamyl transferase (γ-GT), glycated hemoglobin (HbA_{1C}), high sensitivity C-reactive protein (CRP), cotinine and a combination of one or more thereof. Furthermore, the one or more biomarkers may be determined by a method of either immunoassay or enzymatic activity assay.

In terms of the invention, the one or more clinical markers may be selected from the group consisting of age, race, gender, physical activity, medical history, smoking habits, alcohol habits, systolic blood pressure, diastolic blood pressure, perfusion deficits (24h myocardial ischemia events), electrocardiography (ECG) atrial fibrillation, left ventricular hypertrophy (ECG-LVH) and a combination of one or more thereof.

The calculation of the index may be performed using a suitably programmed computer. Furthermore, the index may be a risk score or an equivalent thereof.

Calculation of the index may include the steps of:
- logarithmically transforming the measured levels of one of more biomarkers and one or more clinical markers to generate transformed data;
- multivariate regression model using transformed data to predict stroke risk for all data, 10 training sets and 10 test sets;
- logistic regression using continuous predictors to predict stroke risk in all data and 10 training sets, maximum likelihood estimates (p-value);
- receiver operating characteristics area under the curve (AUC) in all data and 10 training sets predicting stroke risk;
- pearson correlation coefficients for logits of predicted probabilities, based on the stroke risk cut point, with transformed risk components in all data, 10 training and 10 test sets;
- logistic regression using dichotomous predictors to predict stroke risk, presenting maximum likelihood estimates (p-value) for all data and 10 training sets;
- receiver operating characteristics area under the curve (AUC) in all data and 10 training sets predicting stroke risk with dichotomous predictors. Hosmer-Lemeshow tests were performed to test the goodness of fit for the logistic regression risk prediction models (in all participants and 10 training sets);
- logistic regression using dichotomous predictors at baseline and 3-year follow-up to predict stroke risk, presenting maximum likelihood estimates (p-value) for all data; and
- receiver operating characteristics area under the curve (AUC) at baseline and 3-year follow-up predicting stroke risk with dichotomous predictors.

In an embodiment of the invention, the method may include identifying the individual as having an increased likelihood of having a chronic stress and stroke related condition if the generated index is greater than a reference index, and identifying the individual as having a decreased likelihood of having a chronic stress and stroke condition if the generated index is less than the reference index. The reference index may be a standard or a threshold.

Additionally, a method for evaluating the risk of developing chronic stress and stroke in an individual using a computer readable medium having computer executable instructions in a smartphone is described, the method including:
- inputting into a computer the levels of one or more biomarkers measured in the biological sample obtained from the individual;
- inputting into a computer the levels of one or more clinical markers of the individual;
- retrieving a programmatically generated index based on the levels of one or more biomarkers and one or more clinical markers from the computer; and
- displaying the index on a screen of the computer.

In an embodiment, there is provided outputting the index to a user interface device, a local computer system or a remote computer system, or a computer readable storage medium.

In an embodiment, there is provided transmitting, storing, displaying or printing the information related to the likelihood of chronic stress and stroke in an individual.

Furthermore, a use of the above described methods for detecting, identifying, predicting, improving the prediction accuracy of and/or facilitating a therapeutic decision for chronic stress and stroke condition in an individual is described.

These and other aspects of the present invention will now be described in more detail herein and below.

### BRIEF DESCRIPTION OF THE FIGURES

The invention will now be described further, by way of example only, with reference to accompanying figures wherein:

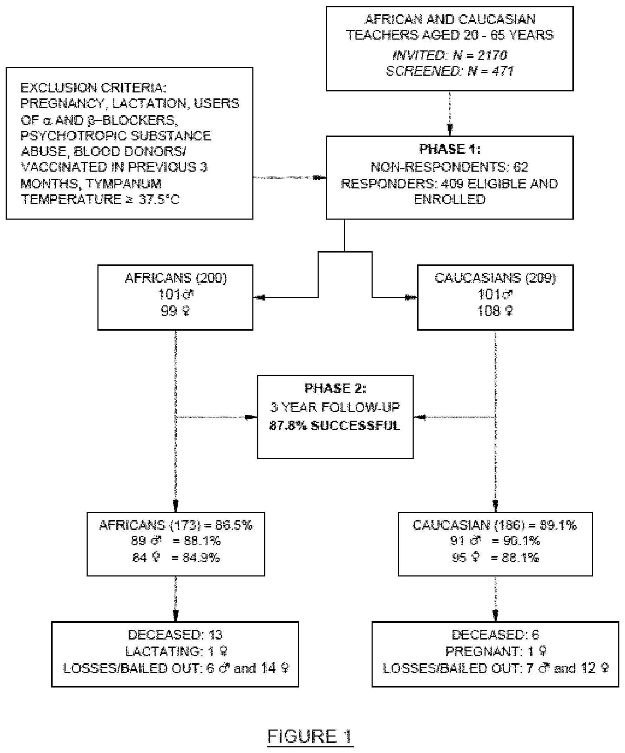

- FIGURE 1: design of the bi-ethnic sex cohort of the Sympathetic Activity and Ambulatory Blood Pressure (SABPA) in Africans prospective study;

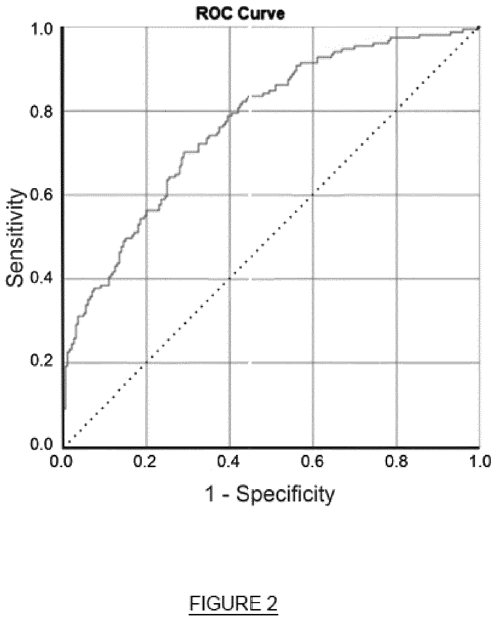

- FIGURE 2: linear regression analyses receiver operating characteristic (ROC) curve depicting chronic stress in the prediction of UCLA 10-year stroke risk score (STRESS^{risk}). The area under the curve, AUC (95% CI) was 0.77 (95% CI 0.72, 0.82) for a positive prediction (85% sensitivity; 48% specificity at a cut point of 0.25) in randomly selected samples (10 training sets, each 60% of population) and 10 test sets (the remaining 40%); and

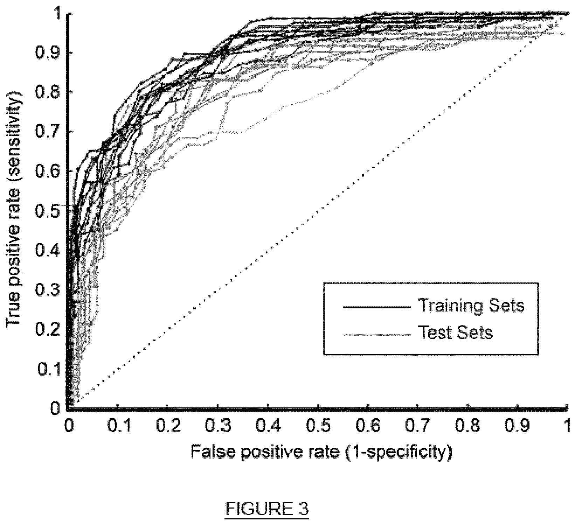

- FIGURE 3: non-linear regression analyses receiver operating characteristic (ROC) curves determined Youden indexes for the probability of chronic stress to predict a UCLA 10-year stroke risk score in the same 10 randomly selected samples (training sets, each 60% of population) and 10 test sets (the remaining 40%) as used in the linear logistic regression analyses.

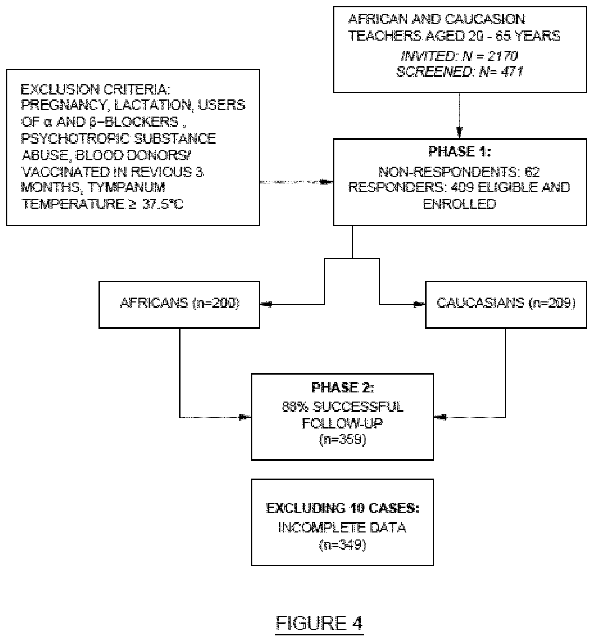

- FIGURE 4: design of the Sympathetic Activity and Ambulatory Blood Pressure (SABPA) in Africans prospective study;

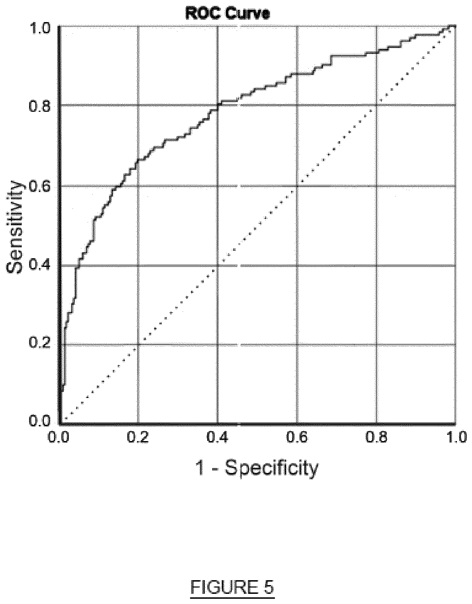

- FIGURE 5: a receiver operating characteristic (ROC) curve depicting a STRESS^{d-RISK} index cut point of 48.43 in the prediction of the probability of the original UCLA 10-year stroke risk. The area under the curve (AUC) (95% CI) was 0.78 (95% CI 0.73; 0.83) for a positive prediction with 81% sensitivity / 59% specificity.

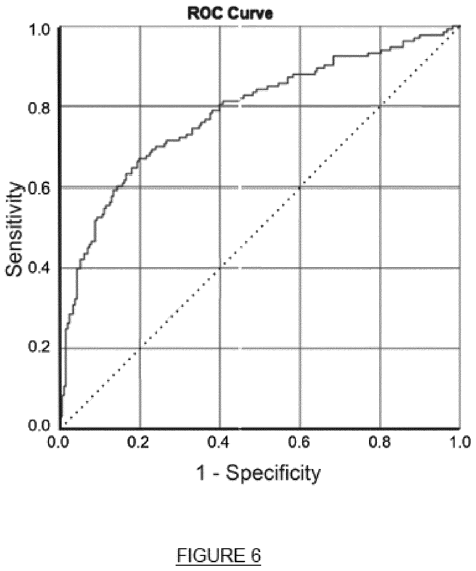

- FIGURE 6: a receiver operating characteristic (ROC) curves of selected input continuous stress biomarkers (V) with dependent variable (Y); a novel 10-year stroke risk marker. A cut point of 46.1% depicted the predicted probability of positives (V). The area under the curve (AUC) (95% CI) was 0.82 (95% CI 0.75; 0.85); p ≤ 0.001 for a positive prediction with 85% sensitivity / 58% specificity.

The foregoing and other objects and features and advantages of the present invention will become more apparent from the following description of certain embodiments of the present invention by way of the following non-limiting examples.

### DETAILED DESCRIPTION OF THE INVENTION

The present invention provides a method for determining a STRESS^{risk} index for the risk of developing chronic stress and ischemic heart disease related stroke and a method for determining a STRESS^{d-RISK} index for the risk of developing chronic stress and diabetes related stroke. Accordingly, biomarkers and clinical markers can be useful in assessing the health state or status of an individual by using a weighted analysis of the levels of one or more biomarkers and one or more clinical markers to generate an index for an individual.

The term "ischemic heart disease" also called coronary heart disease or coronary artery disease has previously been described in Sympathetic Activity and Ambulatory Blood Pressure in Africans (SABPA) study (Malan, *et al.,* 2017). Briefly, ischemia is defined by inadequate blood supply due to narrowing of blood vessels that supply blood and oxygen to the heart muscles. While various factors contribute to the narrowing or constriction of the blood vessels, the interruption in blood supply ultimately results in cellular death of the heart muscles, which lead to complications of the heart during exercise or emotional stress, where an increase in demand of oxygen is experienced, but not adequately met.

The term "stroke" is described as an interruption in the blood supply to the brain, majority (85%) of which is the result of ischemia. Similar to ischemic heart disease, the interruption of blood supply occurs due to occlusions in micro blood vessels, but affects the brain (including retinal vessels) instead of the heart. While diabetes is a separate condition from heart disease, it shares similar threads in that they both affect blood vessels and risk for stroke. Diabetes is a clinical condition present when there is abnormal glucose regulation - where chronically raised levels of glucose is known as hyperglycemia. Moreover, as diabetes is a known independent risk factor for stroke, both heart disease and diabetes share similar characteristics when it comes to management of the diseases.

### EXAMPLE 1

### Identification of Biomarkers

### Study populations

The target population (N = 2170) including urban-dwelling well-educated Black (African) and White African (Caucasian) male and female teachers, enrolled in the 43 schools of the Dr Kenneth Kaunda Education District (Klerksdorp and Potchefstroom), North-West Province, South Africa, and were invited to participate (Figure 1) in the Sympathetic activity and Ambulatory Blood pressure in Africans (SABPA) study cohort. All volunteering teachers had medical aid benefits and were screened to meet study eligibility criteria during the recruitment phase (Figure 1). Those complying formed the respondent group of 409 (Figure 1), and those not complying formed the non-respondent group (N = 62) (Figure 1). The Black teachers preferred to be informed and recruited in separate sex groups and the protocol, especially the amount of blood drawn and hair sampling, was not well received. Time constraints were the main obstacle for participation in the Caucasian teachers' cohort and mixed-sex informed recruitment sessions were not a problem. Data is currently available for 409 teachers of Phase I from which 359 were followed up in Phase II. Exclusion criteria were pregnancy, lactation, tympanum temperature ≥ 37.5°C, the use of psychotropic substances or α- and β-blockers, and blood donors or individuals vaccinated within 3 months prior to data collection.

SABPA analyses were done at the North-West University, Potchefstroom. Facilities and equipment were available to receive and store fasting biological samples at -80°C, before performing analytical assays to detect risk markers.

### Established risk factors

An adaptation of the University of California, Los Angeles 10-year stroke risk composite score (UCLA) [American Heart and Stroke certified UCLA Medical Centre, Primary Stroke Centre, Santa Monica, Los Angeles, USA] was deemed necessary to establish a chronic stress and stroke risk phenotype and said UCLA includes the following variables: individual's medical history (i.e. cardiovascular disease, kidney disease, myocardial infarction, diabetes and hypertension medication usage), demographic and lifestyle factors (age, race, sex, diabetes, smoking, alcohol use and physical activity habits), systolic and diastolic blood pressure, fibrinogen, waist circumference, perfusion deficits (myocardial ischemia), electrocardiography (ECG) atrial fibrillation and ECG left ventricular hypertrophy.

### Biomarkers

Fasting blood samples were collected before 09:00 in the morning to avoid circadian rhythm response fluctuations. Samples were handled according to standardized procedures and frozen at -80°C until required for analysis. For the proposed biochemical analyses, a serum/plasma/urine sample of 500 µl and plasma sample of 200 µl were needed. If serum or plasma was used, the dead volume when using the Hitachi cups for electrochemiluminescence immunoassays on the e411 (ROCHE, Basel, Switzerland) was considered for biochemical analyses.

A registered nurse collected fasting blood samples. All biochemical analyses were done in duplicate on never thawed serum, plasma, urine or saliva samples. Serum cotinine values (indicative of smoking) were derived from a homogeneous immunoassay (Modular ROCHE Automized systems, Basel, Switzerland). Serum and whole blood EDTA samples were analyzed for gamma glutamyl transferase (GGT as indicator of alcohol use), lipids and high sensitivity c-reactive protein (CRP) with an enzyme rated method (Enzymatic colorimetric assay, Cobas Integra 400 plus, ROCHE, Basel, Switzerland. Whole blood EDTA glycated hemoglobin (HbA_{1C}) was analysed with turbidimetric inhibition immunoassays (Cobas Integra 400 Plus, ROCHE Basel, Switzerland). Citrate fibrinogen values were derived by using the viscosity-based clotting method Immuno-turbimetric method 540 nm (Instrument: STA Compact; TAGO Diagnostic, ROCHE, France). Saliva cortisol was analysed with an electrochemiluminescence immunoassay kit (Catalogue number DE2989; Demdemitic Diagnostics GmbH, Kiel-Welsee, Germany). Serum Cortisol, ACTH, and high sensitivity cardiac troponin were determined with an electrochemiluminescence immunoassay (ECLIA), Elecsys 2010 (ROCHE Basel, Switzerland). Values below detectable limit were substituted with lower than detectable values using log-methods. Urine collection was performed overnight, 8 h sampling at baseline and 24 h sampling at follow-up (Malan et al., 2017). At follow-up, participants began and ended sampling with an empty bladder on Day 1. Urine was collected for the next 24 h in a three liter container, washed with 9 ml of 20% HCI (UriSet24, Sarstedi^{®}, Nümbrecht, Germany). Samples were stored at - 80°C until analysis within one year after collection, using the 3-Cat Urine ELISA Fast Track kits (SKU: BA E-6600, LDN, Nordhorn, Germany) where a standard range of 0.5 - 1000 ng/ml was reported. Intra- and inter-assay coefficients for epinephrine were 5.50% and 9.62% respectively and for norepinephrine, 2.70% and 8.59%. Urine creatinine was measured using the enzymatic method (COBAS Integra 400 Plus, ROCHE, Basel; Switzerland). Intra- and inter-assay coefficients for all biochemical analyses were below 10%.

### Cardiovascular measurements

A combined ambulatory blood pressure-electrocardiogram apparatus (Cardiotens CE120^{®}, Meditech, Budapest Hungary) was applied between 07:00 - 09:00 on working days (Monday-Thursday) at the teachers' school of employment. The blood pressure cuff was fitted to the non-dominant arms using an appropriate cuff size. Blood pressure measures were obtained every 30 minutes during the day (08:00-22:00) and hourly during the night (22:00-06:00). Participants continued their usual daily activities and were asked to record occurrences of stress, physical activity, headache, syncope, dizziness, nausea, palpitations, hot flushes and visual disturbances on their ambulatory diary card. The 24h successful inflation rate was 77.9% (±12.9) in "Stressed" individuals and 81.8% (±10.1) in "no-Stressed" individuals. The data were analysed with the CardioVisions 1.19 Personal Edition software (Meditech, Budapest, Hungary). Hypertensive status was classified as 24h SBP ≥ 130 mm Hg and/or DBP ≥ 80 mm Hg (European Society of Cardiology, 2018). Participants resumed their normal school and extracurricular activities till 15:00 and hereafter transported to the North-West University for clinical measures. They fasted from 22:00 till 07:00 when anthropometric measures according to standardized as well as blood samples were collected followed by physical activity measures (Malan et al., 2015).

Silent myocardial ischemia (SMI) events or perfusion deficits: were assessed by two-channel 24h electrocardiogram (ECG) recordings (Cardiotens CE120^{®}, Meditech, Budapest, Hungary) for 20 seconds at 5 minute intervals. Before the start of the ambulatory investigation, the isoelectric reference point (PQ segment), J point, L point (80 ms after the J point), and an ST-segment detection interval of at least 3 mm as the initial ST level, were calculated individually for each patient. An ischemic event was recorded according to the following criteria: horizontal or descending ST-segment depression by at least1 mm; duration of the ST-segment episode lasting ≥ 1 minute, and a ≥ 1-minute interval from the preceding episode. In case of a horizontal or descending ST depression (1 mm-1 minute duration at a 1 minute interval from the preceding episode), an ECG tracing lasting 60 seconds was recorded and an additional blood pressure measurement was automatically initiated by the trigger mechanism of the device.

A resting 12-lead ECG (strip lead II) was used to identify atrial fibrillation cases and which were confirmed by a medical practitioner (NORAV Medical Ltd PC 1200, software version 5.030, Israel). A 12-lead ECG determined ECG left ventricular hypertrophy using strip leads RaVL + SV3 in the calculation of a gender-specific formula, the Cornell product: sum of leads (RaVL+SV3)*QRS > 244mV.ms.

### Retinal vessel analyses (stroke risk marker) (Malan et al., 2020)

Mydriasis was induced in the right eye of the participant by means of a drop containing tropicamide, 1% and benzalkonium chloride 0.01% (m/v). Fundus imaging was performed in a well-controlled light and temperature regulated room with the retinal vessel analyser with a Zeiss FF450^{Plus} camera and the software VesselMap 2, Version 3.02 (Imedos Systems GmbH, Jena, Germany). Retinal vessel calibres were measured as monochrome images by manually selecting first order vessel branches in a measuring zone located between 0.5 and 1.0 optic disc diameters from the margin or the optic disc. Upon selection of the vessel, software automatically delineated the vessels' measuring area. A color image was used as reference to ascertain correct identification of arteries and veins and two experienced scientists agreed on the vessel type before selection. Reproducibility was computed for a randomly selected cohort with a correlation coefficient of 0.84. Diastolic ocular perfusion pressure (DOPP) measures were obtained as hypo-perfusion risk marker in the microvasculature. A local anesthetic drop (Novasine Wander 0.4% Novartis) was inserted in the right eyes in 99 % of all cases to measure intra-ocular pressure (IOP) with the Tono-Pen Avia Applanation Tonometer (Reichert 7-0908, ISO 9001, New York, USA). DOPP was calculated (DBP minus intra-ocular pressure) and hypertensive/diabetic retinopathy was diagnosed by a registered ophthalmologist.

### Statistical Analysis

A risk score for chronic stress and ischemic heart disease related stroke (herein referred to as STRESS^{risk} index) may reflect chronic stress and stroke risk. The statistical software packages used were Statistica version 13.3 (TIBCO Software Inc., Palo Alto, USA, 2018); IBM SPSS version 23 statistical and SAS^{®} 9.4 (Statistical Analysis System). Variables with skewed distributions were log-transformed. The statistical significance level was set at p ≤ 0.05 (two-tailed).

The STRESS^{risk} index was determined as follows:
A multiple stepwise linear regression of biomarkers and risk factors (transformed to be normally distributed) of the UCLA was performed.

The Receiver Operating Characteristic (ROC) analysis is commonly used to assess the difference between two distributions (binary classification) at all classification thresholds. The ROC space consists of a plot of a continuous system represented by a (ROC) curve, created by plotting the true positive rate against the false positive rate, and the area under the ROC curve (AUC) is employed as a measure of the performance of the predictions made from the classification system across the different thresholds. The STRESS^{risk} index, for three or more possible combination of continuous biomarkers, was determined as the AUC as a maximum (Youden index: sensitivity + specificity - 1) when discriminating the positive and negatives of the UCLA composite dichotomous marker (range from 2-30 %) denoted as Y. The Youden index is a method that finds the point on the ROC curve farthest from the change classification, and is used to identify a "optimal" cut-off value. Here, the term "optimal" refers to the cut point that maximizes correct classifications and/or minimizes incorrect classifications. Accordingly, an optimal cut-off value for the STRESS^{risk} index was used and denoted as biomarker V.

### Validation of the biomarker V:

Here Y was used as the dependent variable and V as predicted probability of positives using a logistic regression on the selected input continuous biomarkers and confounding risk factors.

To discriminate the AUC between the positives and negatives of Y using the predicted probability of positives and also the sensitivity and specificity of correct predictions were used as diagnostics for predictive validity. An optimal cut-off value was further determined for V using the ROC analysis.

Non-linear regression model, that includes neural networks, was compared with the logistic regression model. The maximum of the Youden index (sensitivity + specificity - 1) was determined using the ROC curves, with the non-linear regression analyses substantiating the novel functional relationship between the models using multilayer perceptron with 2 layers and trained with Bayesian regularization. Hidden layers have tansig functions and the output layer is linear with 10 bootstrap repetitions.

Once the networks were optimized, they were used to extract the required functional relationships with the UCLA stroke risk scores.

### Results

**Table 1: Clinical characteristics of a chronic stress and ischemic heart disease related stroke risk phenotype.**

| | Stressed (N=236) | Non-Stressed (N=123) | P-values |
|---|---|---|---|
| Age, yrs | 44.5 (39.0-51.0) | 47.0 (41.0-54.0) | 0.04 |
| Women, n (%) | 88 (52.1) | 82 (47.4) | 0.39 |
| Urban living, years | 31.8 (19.0-45.0) | 20.5 (10.0-30.0) | < 0.001 |
| Cotinine, ng/ml | 0.01 (0.01-15.51) | 0.01 (0.01-0.01) | 0.33 |
| GGT, U/I | 43.5 (28.4-74.4) | 18.0 (12.0-28.0) | < 0.001 |
| Physical activity, kcal/24h | 2584.6 (2185.9-3118.1) | 2968.0 (2370.0-3540.7) | < 0.001 |
| Waist circumference, cm | 98.2 (88.7, 106.3) | 83.3 (74.6, 93.4) | <0.001 |

| **Ischemic heart disease and stroke risk markers** | | | |
|---|---|---|---|
| Thyroid stimulating hormone, µIU/ml | 1.8 (1.3-2.5) | 2.1 (1.4-2.9) | 0.01 |
| Intra-ocular pressure (mmHg) | 16 (4) | 15 (4) | 0.044 |
| Retinal artery caliber (MU) | 148.5 (11.1) | 154.1 (13.4) | <0.001 |
| Retinal vein caliber (MU) | 243.2 (20.0) | 240.6 (20.2) | 0.267 |
| Retinopathy, n (%) | 134 (57) | 55 (45) | 0.024 |
| Cardiac Troponin T, ng/L | 4.2 (3.1-5.5) | 4.9 (3.2-6.9) | 0.05 |
| Cholesterol, mmol/I | 4.5 (3.8-5.5) | 5.5 (4.7-6.4) | < 0.001 |
| CRP:Fibrinogen, g/L:mg/L | 1.4 (0.7-2.6) | 0.5 (0.4-1.2) | <0.001 |
| 24h SBP, mm Hg | 131 (122-143) | 124 (116-130) | <0.001 |
| 24h DBP, mm Hg | 82 (77-90) | 77 (71-82) | <0.001 |
| 24h Heart rate, bpm | 79 (73-86) | 74 (68-81) | <0.001 |
| 24h Hypertension, n (%) | 176 (75) | 19 (16) | <0.001 |
| 24h urinary NE:Cr | 18.8 (11.6-29.8) | 24.8 (13.2-38.9) | 0.07 |
| 24h urinary E:Cr | 2.9 (1.6-2.9) | 2.9 (1.6-4.7) | 0.36 |

| **Medications, n (%)** | | | |
|---|---|---|---|
| Statins | 2 (1.2) | 6 (3.5) | 0.16 |
| Aspirin | 4 (2.4) | 9 (5.2) | 0.17 |
| ACE inhibitors | 19 (11.2) | 3 (1.7) | <0.001 |
| Angiotensin II blockers | 1 (0.6) | 1 (0.6) | 0.99 |
| Diuretics | 23 (13.6) | 8 (4.6) | <0.001 |
| Calcium channel blockers | 13 (7.7) | 1 (0.6) | 0.001 |
| Beta blockers | 5 (3.0) | 1 (0.6) | 0.09 |
| Alpha blockers | 0.0 | 0.0 | - |
| Independent t-tests were used to compare "Stressed" vs. "non-Stressed" groups. Values are mean (±SD), median (± interquartile range/IQR) or frequencies (%). Where: GGT, gamma glutamyl transferase; CRP, C-reactive protein; HDL, high density lipoproteins; HRV, heart rate variability measuring the standard deviation of the 12-lead ECG RR interval; NE:Cr, norepinephrine creatinine ratio; E:Cr, epinephrine creatinine ratio. Hypertensive status classified as 24h SBP ≥ 130 mm Hg and/or DBP ≥ 80 mm Hg. | | | |

### EXAMPLE 2

The retinal vessels offer an easily accessible view of the vasculature, which might reflect emotional stress pathology and stroke risk. As the retina shares embryonic origins with the brain, with similar anatomy and blood-barrier physiology; it is of particular interest as a marker of cerebrovascular and neurodegenerative disease.

The STRESS^{RISK} index was used to assess "Stressed" vs. "non-Stressed" groups, independent of race or sex. Findings showed that behavior and biological processes are tightly interlinked in the brain-retina-heart axis. For example, cardiac injury and stroke risk markers (including retinal arterial narrowing) reflected inflammation and oxygen perfusion deficits, of which is associated with increased risk for stroke in "Stressed" individuals. Furthermore, arterial narrowing and vein widening in the retina were associated with decreased glial cell functioning in these "Stressed" individuals (Malan et al., 2020), which resembles findings previously shown in the prefrontal cortex of suicide cases and severely depressed patients. As a consequence, this may also have debilitating effects on retinal ganglion cell health and visual function.

The emerging metabolic perturbation and endothelial dysfunction observed in the "Stressed" individuals is determined as the chronic stress and diabetes related stroke risk phenotype (herein referred to as STRESS**^{d-RISK}**).

### Study Population

The SABPA prospective cohort study was used as complete data source (n=349) (Figure 4) and all individuals participated at baseline and at 3-year follow-up. The rationale for the selection of the participants was to obtain a sample from a homogenous working environment with similar socio-economic status. Seasonal changes were avoided and extensive clinical assessments were to be performed in a well-controlled temperature and light setting environment. Exclusion criteria at baseline were pregnancy, lactation, tympanum temperature ≥ 37.5°C, the use of psychotropic substances or α- and β-blockers and blood donors or individuals vaccinated within 3 months prior to data collection. Participants were fully informed about the objectives and procedures prior to recruitment and provided written, informed consent.

### Biochemical analyses

Participants were in a semi-recumbent position for at least 30 minutes before 09:00 in both study phases. A registered nurse obtained fasting blood samples from the antebrachial vein branches of the dominant arm of each participant with a winged infusion set. All blood samples were handled according to standardized procedures and stored at -80°C until analyses. All biochemical analyses were done in duplicate on never thawed serum/plasma samples. Serum cotinine values (indicative of smoking) were derived from a homogeneous immunoassay (Modular ROCHE Automized systems, Basel, Switzerland). Serum and whole blood EDTA samples were analyzed for gamma glutamyl transferase (GGT as indicator of alcohol use), lipids and high sensitivity c-reactive protein (CRP) with an enzyme rated method (Enzymatic colorimetric assay, Cobas Integra 400 plus, ROCHE, Basel, Switzerland. Total Insulin-like growth factor-1 was determined in serum using an immunoradiometric assay (IRMA) from Immunotech, Beckman Coulter (A15729). With an inter-assay percentage coefficient of variation of 4.49 and an intra-assay percentage coefficient of variation 2.92. Whole blood EDTA glycated hemoglobin (HbA_{1C}) were analysed with turbidimetric inhibition immunoassays (Cobas Integra 400 Plus, ROCHE Basel, Switzerland). The American Diabetes Foundation guidelines were used to define pre-diabetes status as ≥5.7%; diabetes as HbA_{1C} ≥ 6.5%; and HOMA-IR by using the following formula: fasting glucose x fasting insulin/405 [normal IR, <3, moderate IR, 3-5; and severe IR, >5].

### Statistical Analysis

The statistical software packages used were Statistica version 13.3 (TIBCO Software Inc., Palo Alto, USA, 2018); IBM SPSS version 23 statistical and SAS^{®} 9.4. The SABPA prospective cohort study was used as complete data source (n=349) and all individuals participated at baseline and at 3-year follow-up.

The following statistical analyses were carried out in order to validate chronic stress and determine the probability of diabetes related stroke risk.

Variables with skewed non-normal distributions were logarithmic transformed. The statistical significance level was set at p ≤ 0.05 (two-tailed).

The validated chronic stress risk biomarkers predictive of stroke were used to establish a chronic stress and diabetes related stroke risk phenotype.

An adaptation of the UCLA was used to determine the risk of chronic stress and diabetes related stroke risk in an individual, where the 9 independent markers of said UCLA include: age, sex, systolic blood pressure, self-reported use of hypertensive drugs, diabetes, smoking habit, perfusion deficits (myocardial ischemia), ECG atrial fibrillation and ECG-LVH. The UCLA includes self-reported measures for use of hypertensive drugs, diabetes and smoking habit, which may not be reliable due to concerns relating to biases and other limitations (Epel et al., 2018; Malan et al., 2017; Malan et al., 2020). Accordingly, these self-reported variables were replaced with quantitative validated markers, i.e. HbA_{1C} ≥ 6.5% as a marker for diabetes (American Diabetes Association 2019) and nicotine metabolite (i.e. cotinine ≥ 14ng/ml) as a marker for smoking. In addition, alcohol abuse, did not form part of the UCLA and thus the liver enzyme, GGT, was used as a marker for alcohol abuse (Hastedt et al., 2016; Enhörning & Malan 2019). Apart from high blood pressure, habitual consumption of large amounts of alcohol is one of the most important risk factors for stroke (Solveig et al., 2018), contributing to more than 50% of all stroke cases in the United Kingdom. Furthermore, a previous study has shown that alcohol abuse was associated with high blood pressure (Hamer et al., 2011), which is also related with the increased risk of developing ischemic heart disease (Oosthuizen et al., 2016; Wentzel et al., 2018) as well as the onset of stroke (Mostofsky et al., 2010).

Standardized values of the adapted UCLA were determined by using principal component analysis at baseline. The first principal component scores were computed as a weighted mean of standardized variables with determined weights reflecting 7 component loadings [cotinine, GGT, diabetes (HbA1C ≥ 6.5%), systolic blood pressure, perfusion deficits (myocardial ischemic events), atrial fibrillation and ECG-LVH]. The first principal component scores then had a mean of 0 and standard deviation of 1. To obtain a convenient index, the component score values were multiplied by 10 and increased by 50. A so-called T-index, having a mean of 50 and a standard deviation of 10 and lies between 0 and -100, was denoted as the STRESS^{d-RISK} index.

A cut-point for STRESS^{d-RISK} index (discriminatory analyses) was determined by conducting the ROC analysis using the cut-off of the original UCLA 10-year stroke risk score (Figure 5). This discriminated between the positive and negative data using the STRESS^{d-RISK} index and also the sensitivity, specificity and percentage of correct predictions for obtaining the cut point. The dichotomous variable, which discriminates between those respondents who are at risk (when above the cut point) and those not at risk (below the cut point), is denoted by Y. The AUC was computed with 95% confidence limits.

Multivariate linear regression analysis was applied in a model using logarithmic transformed predictors to validate the chronic stress and diabetes related stroke risk phenotype (i.e. STRESS^{d-RISK} index) in a complete dataset of N=349, by using subsets of 10 training sets (N=209, i.e. each 60% of population) and 10 test sets (the remaining 40%). Regression coefficient estimates and p-values were determined in all regressions.

Logistic linear regression was applied in a model by using logarithmic transformed predictors in all data as before using the aforementioned dependent variable Y. To validate the logistic linear regression model, model fitting was repeated on 10 randomly selected samples (training sets, each 60% of population) and 10 test sets (the remaining 40%). The maximum likelihood estimates of regression coefficients were obtained in all these regressions to predict the probability of risk for chronic stress and diabetes related stroke.

To discriminate between the positive and negatives of the novel stroke risk marker, the dichotomous marker Y of the STRESS^{d-RISK} index was used. Using a logistic regression analysis, Y was used as the dependent variable and V, contained the selected input continuous stress biomarkers, as predictors of positives. An optimal cut-off value was further determined for V using ROC analysis (Figure 6).

The AUC in the ROC analysis on V (using Y) and the sensitivity, specificity and percentage correct predictions at the cut-off value were considered as diagnostics for predictive validity of V. Hosmer-Lemeshow tests were performed to test the goodness of fit for the logistic regression risk prediction models (in all participants, 10 training and 10 test sets).

### Results

**Table 2: Clinical characteristics of a chronic stress and diabetes related stroke risk phenotype.**

| | **Stress (n=159)** | **no-Stress (n=105)** | **P values** |
|---|---|---|---|
| Age (years) | 46.2 (±9.1) | 45.4 (±9.2) | 0.501 |
| Ethnicity: Blacks | 97 (61) | 21 (20) | <0.001 |
| Sex: Men | 114 (72) | 29 (28) | <0.001 |

| *Diabetes and stroke risk markers* | | | |
|---|---|---|---|
| Total energy expenditure (kcal/24h) | 3145 (±1706) | 2744 (±807) | 0.015 |
| Smoking status | 45 (28) | 6 (6) | <0.001 |
| Alcohol abuse | 90 (57) | 4 (4) | <0.001 |
| Ethnic-specific central obesity | 105 (66) | 54 (34) | 0.018 |
| Dyslipidemia | 77 (48) | 34 (32) | 0.011 |
| Low grade inflammation | 86 (54) | 40 (38) | 0.011 |
| Insulin-like growth factor-I (ng/ml) | 0.9 (0.1, 30.2) | 2.5 (0.4, 64.9) | 0.031 |
| Pre-diabetes | 108 (68) | 18 (17) | <0.001 |
| Diabetes | 20 (13) | 0 (0) | <0.001 |
| Severe HOMA-IR | 52 (33) | 11 (11) | <0.001 |
| Perfusion deficits-DOPP (mmHg) | 73 (±11) | 66 (±11) | <0.001 |
| Diabetes/Hypertensive retinopathy | 98 (62) | 46 (44) | <0.001 |
| 24h Hypertension | 118 (74) | 32 (31) | <0.001 |

| *Self-reported medication* | | | |
|---|---|---|---|
| Diabetes | 8 (5) | 0 (0) | 0.020 |
| Hypertension | 48 (30) | 18 (17) | 0.021 |
| Anti-depressant | 1 (1) | 1 (1) | 0.767 |
| | | | |

Values are presented as mean (±SD), or N (%), and/or median (± 95% interquartile range). Abbreviations: Smoking status, cotinine, ≥14ng/ml; Alcohol abuse, GGT ≥ 55 U/L (Oosthuizen et al., 2016); Ethnic-specific central obesity cut points (Malan et al., 2015); Dyslipidemia, total cholesterol:high density lipoprotein cholesterol ≥ 5.1; Low grade inflammation (CRP ≥ 3ng/ml); Prediabetes, HbA1C ≥ 5.7%; Diabetes, HbA1C ≥ 6.5%; Severe HOMA-IR, homeostasis model of insulin resistance/IR assessment (>5); DOPP, Diastolic ocular perfusion pressure; 24h Hypertension, SBP ≥ 130 and/or DBP ≥ 80 mmHg;

### REFERENCES

Enhörning, S., Malan, L. (2019). Copeptin relates to a fatty liver and measures of obesity in a South African population with mixed ethnicities. Endocrine 65, 304-311.
Epel, S.E., McEwen, B., Seeman, T., Matthews, K., Castellazo, G., Brownell, K.D., Bell, J., Ickovics, J.R. (2000). Stress and Body Shape: Stress-Induced Cortisol Secretion Is Consistently Greater Among Women With Central Fat. Psychosom Med 62, 623-632.
Hamer, M., Malan, L., Malan, N.T., Schutte, A.E., Huisman, H.W., van Rooyen, J.M., Schutte, R., Fourie, C.M.T., Seedat, Y.K. (2011). Conventional and behavioural risk factors and sub-clinical atherosclerosis in black and white Africans: The SABPA study. Atherosclerosis 215, 237-242.
Hastedt M, Büchner M, Rothe R, Gapert R, Herre S, Krumbiegel F, Tsokos M, Kienast T, Heinz A, Hartwig S. (2013). Detecting alcohol abuse: traditional blood alcohol markers compared to ethyl glucuronide (EtG) and fatty acid ethyl esters (FAEEs) measurement in hair. Forensic Sci Med Pathol 9(4), 471-477.
Malan, L., Hamer, M., Frasure-Smith, N., Steyn, H.S., Malan, N.T. (2015). COHORT PROFILE: Sympathetic activity and Ambulatory Blood Pressure in Africans (SABPA) Prospective Cohort Study. Int J Epidem 44(6), 1814-1822.
Malan, L., Hamer, M., von Känel, R., Lambert, G.W., Delport, R., Steyn, H.S., Malan, N.T. (2017). Chronic defensiveness and neuroendocrine dysregulation reflect a novel cardiac Troponin T cut point: the SABPA study: Psychoneuroendocrinology 85, 20-27.
Malan, L., Hamer, M., von Känel, R., van Wyk, R.D., Wentzel, A., Steyn, H.S., van Vuuren P, Malan NT. (2020). Retinal-glia ischemia and inflammation induced by chronic stress: the SABPA study. Brain, Behav Imm - Health 2, 100027.
Mirza, S.S., de Bruijn, R.F.A.G., Koudstaal, P.J., van den Meiracker, A.H., Franco, O.H., Hofman, A., Tiemeier, H., Ikram, A. (2016). The N-terminal pro B-type natriuretic peptide, and risk of dementia and cognitive decline: a 10-year follow-up study in the general population. J Neurol Neurosurg Psych, 87, 356-362.
Mostofsky, E., Burger, M.R., Schlaug, G., Mukamal, K.J., Rosamond, W.D., Mittleman, M.A. (2010). Alcohol and acute ischemic stroke onset: the stroke onset study. Stroke 41(9), 1845-1849.
Oosthuizen, W., Malan, L., Scheepers, J.D., Cockeran, M., Malan, N.T. (2016). The defense response and alcohol intake: A coronary artery disease risk? The SABPA Study. Clin Exp Hypertens 38(6), 526-532.
Solveig, A., Cunningham, M., Judd, S.E., Matz, L.M., Kabagambe, E.K., Moy, C.S., Howard, V.J. (2018). Alcohol Consumption and Incident Stroke Among Older Adults. J Gerontol B Psychol Sci Soc Sci 73(4), 636-648.
von Känel, R., Hamer, M., Van Der Westhuizen, F.H., Malan, N.T., Malan, L. (2014). Leukocyte telomere length and hemostatic factors in a South African cohort: The SABPA Study. Thromb Hemo 12,1975-1985**.**
Wentzel A, Malan L, Scheepers JD, Malan NT. (2018). QTc prolongation, increased NT-proBNP and pre-clinical myocardial wall remodelling in excessive alcohol consumers: The SABPA study. Alcohol 68, 1-8.
Wesseling, K.H., Jansen, J.R.C., Settels, J.J., Schreuder, J.J. (1993). Computation of aortic flow from pressure in humans using a nonlinear, three-element model. J App Physiol 74, 2566-2573.

## Claims

1. A computer-implemented method for determining a STRESS^{risk} index for the risk of developing chronic stress and ischemic heart disease related stroke including:
i) Performing a multiple stepwise linear regression of biomarkers and risk factors, both transformed to be normally distributed, of an adapted UCLA score, wherein said UCLA score is a 10-year stroke risk composite score of the University of California, Los Angeles (UCLA) and, which includes the variables: individual's medical history; i.e., cardiovascular disease, kidney disease, myocardial infarction, diabetes and hypertension medication usage; demographic and lifestyle factors; i.e., age, race, sex, diabetes, smoking, alcohol use and physical activity habits; systolic and diastolic blood pressure; fibrinogen; waist circumference; perfusion deficits, i.e., myocardial ischemia; electrocardiography atrial fibrillation; and electrocardiography left ventricular hypertrophy;
wherein the biomarkers and risk factors have been determined by obtaining a fasting biological sample i.e., blood, urine, and saliva from individuals; measuring the levels of the following biomarkers in the biological samples obtained from the individuals and measuring the levels of the following clinical markers of the individuals; wherein the biomarkers are serum cotinine values derived from a homogeneous immunoassay, gamma-glutamyl transferase (γ-GT), lipids and high sensitivity c-reactive protein (CRP) analyzed with an enzyme rated method, whole blood EDTA glycated hemoglobin (HbA_{1C}) analyzed with turbidimetric inhibition immunoassay, citrate fibrinogen values derived by viscosity-based clotting method Immuno-turbimetric method 540 nm, saliva cortisol analyzed by an electrochemiluminescence immunoassay kit, serum cortisol determined with an electrochemiluminescence immunoassay, adrenocorticotrophic hormone (ACTH) determined with an electrochemiluminescence immunoassay, high sensitivity cardiac troponin determined with an electrochemiluminescence immunoassay, wherein acidified urine was collected overnight for urinary norepinephrine and epinephrine analysis using a 3-Cat Urine ELISA FAST TRACK kit, and wherein urine creatinine was measured using the enzymatic method; wherein the clinical markers are systolic blood pressure (SBP), diastolic blood pressure (DBP) both measured with a combined ambulatory blood pressure-electrocardiogram apparatus, silent myocardial ischemia (SMI) events or perfusion deficits assessed by two channel 24h electrocardiogram recordings, electrocardiography (ECG), atrial fibrillation identified by a resting 12-lead ECG, ECG left ventricular hypertrophy (ECG-LVH) determined by a 12-lead ECG, retinal vessel calibers measured as monochrome images using a retinal vessel analyzer and vessel-map-software, intra-ocular pressure (IOP) measured with a Applanation Tonometer, and diastolic ocular perfusion pressure (DOPP) calculated from DBP minus IOP;
ii) Performing a Receiver Operating Characteristic (ROC) analysis to assess the difference between the distribution of biomarkers and the distribution of risk factors at all classification thresholds;
iii) Determining the STRESS^{risk} index for three or more possible combinations of continuous biomarkers as an area under the curve (AUC) as a maximum of the ROC analysis, when discriminating the positive and negatives of the UCLA composite dichotomous biomarker denoted as Y, wherein the STRESS^{risk} index AUC was 0.77 (95% Cl 0.72, 0.82) for a positive prediction (85% sensitivity, 48% specificity);
iv) Determining a cut-off value by the Youden index for the STRESS^{risk} index that maximizes correct classifications and/or minimizes incorrect classifications and denotes the combination of biomarkers at the determined optimal cut-off value as biomarker V;
v) Validating biomarker V by using Y as the dependent variable and V as the predicted probability of positives using a logistic regression on the selected input continuous biomarkers and confounding risk factors;
vi) Discriminating the AUC between the positives and negatives of Y using the predicted probability of positives and using the sensitivity and specificity of correct predictions as diagnostic for predictive validity;
vii) Determining an optimal cut-off value for V using ROC analysis;
viii) Using a non-linear regression model that includes neural networks as comparison to the logistic regression model;
ix) Determining the maximum of the Youden index using the ROC curves with the non-linear regression analysis substantiating the functional relationship between the models using multilayer perceptron with two layers and trained with Bayesian regularization, wherein hidden layers have tansig functions and the output layer is linear with ten bootstrap repetitions;
x) Optimizing the network and extracting the functional relationship with the UCLA stroke risk scores.

2. A computer-implemented method for determining a STRESS^{d-RISK} index for the risk of developing chronic stress and diabetes related stroke including:
i) Using the STRESS^{risk} index determined according to claim 1 to establish the STRESS^{d-RISK} index;
ii) Performing a statistical analysis of biomarkers and risk factors, wherein variables with skewed non-normal distributions were logarithmically transformed;
wherein the biomarkers and risk factors are the biomarkers and risk factors of the adapted UCLA score, with the following nine independent markers: age, sex, systolic blood pressure, self-reported use of hypertensive drugs, diabetes, smoking habit, perfusion deficits, ECG atrial fibrillation and ECG-LVH, wherein the self-reported variables were replaced with quantitative markers, i.e. HbA1C ≥ 6.5% as a marker for diabetes, and nicotine metabolite cotinine ≥ 14 ng/ml as a marker for smoking, wherein in addition the liver enzyme gamma glutamyl transferase (GGT) was used as a marker for alcohol abuse;
wherein the quantitative markers were determined by measuring in fasting blood samples: serum cotinine values determined by a homogeneous immunoassay, gamma glutamyl transferase (GGT) measured in serum, lipids and high sensitivity c-reactive protein measured in whole blood EDTA samples with an enzymatic colorimetric assay, total insulin-like growth factor-1 determined in serum using an immunoradiometric assay, and whole blood EDTA glycated hemoglobin (HbA1C) analyzed in with turbidimetric inhibition immunoassays;
iii) Determining standardized values of the adapted UCLA score by principal component analysis at baseline;
iv) Computing the first principal component scores as a weighted mean of standardized variables with determined weights reflecting the following seven component loadings: cotinine, GGT, diabetes defined as HbA1C ≥ 6.5%, systolic blood pressure, perfusion deficits, atrial fibrillation and ECG-LVH;
v) Determining the STRESS^{d-RISK} index by multiplying the component scores values by ten and increasing it by fifty, such that the mean of the STRESS^{d-RISK} index is 50 and its standard deviation lies between 0 and minus 100;
vi) Determining a cut-point for the STRESS^{d-RISK} index by conducting a ROC analysis using the cut-off determined in claim 1 iv), to discriminate between the positive and negative data and the sensitivity, specificity and percentage of correct predictions;
vii) Denoting the dichotomous variable, which discriminates between those respondents above the cut point and those below the cut point, as Y, wherein the STRESS^{d-RISK} index AUC was 0.78 (95% Cl 0.73, 0.83) for a positive prediction (81% sensitivity, 59% specificity);
viii) Validating the STRESS^{d-RISK} index by applying multivariate linear regression analysis in a model using logarithmic transformed predictors in a complete dataset of N=349, by using subsets of 10 training sets with each 60% of population and 10 test sets with the remaining 40% of population;
ix) Applying logistic linear regression in a model by using logarithmic transformed predictors in all data as in viii) using variable Y as in vii);
x) Validating the logistic linear regression model by repeating model fitting on 10 randomly selected samples and 10 test sets;
xi) Predicting the probability of risk for chronic stress and diabetes related stroke by obtaining the maximum likelihood estimates of regression coefficients of all regressions;
xii) Using the dichotomous marker Y of the STRESS^{d-RISK} index as in vii) to discriminate between the positive and negatives of the markers of xi);
xiii) Using a logistic regression analysis wherein Y is used as the dependent variable and V contains the selected input continuous stress biomarkers as predictors of positives, wherein the STRESS^{d}-^{risk} index AUC was 0.82 (95% Cl 0.75, 0.85) for a positive prediction (85% sensitivity, 58% specificity);
xiv) Determining an optimal cut-off value for V using ROC analysis;
xv) Using the area under the curve (AUC) in the ROC analysis on V, using Y, and the sensitivity, specificity and percentage correct predictions at the cut-off value as diagnostics for the predictive validity of V; and
xvi) Performing a Hosmer-Lemeshow test for testing the goodness of fit for the logistic regression risk prediction models in all participants, training and test sets.

## Patentansprüche

1. Computerimplementiertes Verfahren zum Bestimmen eines Stress-Risiko-Index (STRESS^{risk} index) für das Risiko der Entwicklung von chronischem Stress und mit einer ischämischen Herzkrankheit in Beziehung stehendem Schlaganfall, aufweisend:
i) Ausführen einer mehrstufigen linearen Regression von Biomarkern und Risikofaktoren, die beide so transformiert wurden, dass sie normalverteilt sind, eines angepassten UCLA-Scores, wobei der UCLA-Score ein kombinierter 10-Jahres-Schlaganfallrisiko-Score der University of California, Los Angeles (UCLA) ist und die folgenden Variablen aufweist: Anamnese des Patienten, d.h. Herz-Kreislauf-Erkrankung, Nierenerkrankung, Myokardinfarkt, Diabetes und Einnahme blutdrucksenkender Medikamente, demografische Faktoren und Lebensstilfaktoren, d.h. Alter, ethnische Zugehörigkeit, Geschlecht, Diabetes, Rauchgewohnheiten, Alkoholkonsum und körperliche Aktivität, systolischer und diastolischer Blutdruck, Fibrinogen, Taillenumfang, Durchblutungsstörungen, d.h. Myokardischämie, Vorhofflimmern im Elektrokardiogramm und linksventrikuläre Hypertrophie im Elektrokardiogramm,
wobei die Biomarker und Risikofaktoren durch Entnahme einer biologischen Nüchternprobe, d.h. Blut, Urin und Speichel, von Personen, Messen der Konzentrationen der folgenden Biomarker in den von den Personen entnommenen biologischen Proben und Messen der Konzentrationen der folgenden klinischen Marker der Personen bestimmt worden sind, wobei die Biomarker Serum-Cotinin-Werte sind, die von einem homogenen Immunoassay hergeleitet werden, Gamma-Glutamyltransferase (γ-GT), Lipide und hochsensitives c-reaktives Protein (CRP), analysiert mit einer enzymbasierten Methode, Vollblut-EDTA-glykiertes Hämoglobin (HbA_{1C}), analysiert mit einem turbidimetrischen Hemmungs-Immunoassay, Citrat-Fibrinogenwerte, hergeleitet durch ein viskositätsbasiertes Gerinnungsverfahren, immunoturbimetrisches Verfahren 540 nm, Speichelcortisol, analysiert durch ein Elektrochemilumineszenz-Immunoassay-Kit, Serumcortisol, bestimmt durch ein Elektrochemilumineszenz-Immunoassay, adrenokortikotropes Hormon (ACTH), bestimmt durch ein Elektrochemilumineszenz-Immunoassay, hochsensitives kardiales Troponin, bestimmt durch ein Elektrochemilumineszenz-Immunoassay, wobei über Nacht angesäuerter Urin für die Analyse von Noradrenalin und Adrenalin im Urin unter Verwendung eines 3-Cat Urine ELISA FAST TRACK-Kit gesammelt wurde, und wobei Kreatinin im Urin unter Verwendung der enzymatischen Methode gemessen wurde, wobei die klinischen Marker der systolische Blutdruck (SBP) und der diastolische Blutdruck (DBP) sind, die beide mit einem kombinierten ambulanten Blutdruck-Elektrokardiogrammgerät gemessen werden, stille Myokardischämie- (SMI) Ereignisse oder Perfusionsdefizite, die durch Zweikanal-24-Stunden-Elektrokardiogrammaufzeichnungen beurteilt werden, Elektrokardiographie (EKG), Vorhofflimmern, das durch ein Ruhe-12-Kanal-EKG identifiziert wurde, eine durch ein 12-Kanal-EKG bestimmte linksventrikuläre Hypertrophie (ECG-LVH), Kaliber der Netzhautgefäße, die als monochrome Bilder unter Verwendung eines Netzhautgefäßanalysators und einer Gefäßkartierungssoftware gemessen wurden, ein durch ein Applanationstonometer gemessener Augeninnendruck (IOP) und ein von DBP minus IOP berechneter diastolischer okulärer Perfusionsdruck (DOPP);
ii) Ausführen einer ROC-, Receiver Operating Characteristic, Analyse zum Bewerten der Differenz zwischen der Verteilung von Biomarkern und der Verteilung von Risikofaktoren bei allen Klassifizierungsschwellenwerten;
iii) Bestimmen des Stress-Risiko-Index für drei oder mehr mögliche Kombinationen von kontinuierlichen Biomarkern als eine Fläche unter der Kurve (AUC) als ein Maximum der ROC-Analyse, wenn zwischen positiven und negativen Daten des als Y bezeichneten kombinierten dichotomen UCLA-Biomarkers unterschieden wird, wobei der Stress-Risiko-Index AUC für eine positive Vorhersage 0,77 (95% CI 0,72, 0,82) betrug (85% Sensitivität, 48% Spezifität);
iv) Bestimmen eines Cut-off-Wertes durch den Youden-Index für den Stress-Risiko-Index, der korrekte Klassifizierungen maximiert und/oder falsche Klassifizierungen minimiert und die Kombination von Biomarkern bei dem bestimmten optimalen Cut-off-Wert als Biomarker V markiert;
v) Validieren des Biomarkers V unter Verwendung von Y als die abhängige Variable und V als die vorhergesagte Wahrscheinlichkeit von positiven Daten unter Anwendung einer logistischen Regression auf die ausgewählten kontinuierlichen Eingangsbiomarker und Stör-Risikofaktoren;
vi) Unterscheiden der AUC zwischen den positiven und negativen Daten von Y unter Verwendung der vorhergesagten Wahrscheinlichkeit von positiven Daten und unter Verwendung der Sensitivität und Spezifität korrekter Vorhersagen als Diagnose für prädiktive Validität;
vii) Bestimmen eines optimalen Cut-off-Wertes für V unter Verwendung einer ROC-Analyse;
viii) Verwenden eines nichtlinearen Regressionsmodells, das neuronale Netzwerke enthält, als Vergleich zum logistischen Regressionsmodell;
ix) Bestimmen des Maximums des Youden-Index unter Verwendung der ROC-Kurven durch die nichtlineare Regressionsanalyse, die die funktionale Beziehung zwischen den Modellen unter Verwendung eines mehrschichtigen Perzeptrons mit zwei Schichten und trainiert mit Bayes'scher Regularisierung substantiiert, wobei verborgene Schichten Tansig-Funktionen haben und die Ausgabeschicht linear mit zehn Bootstrap-Wiederholungen ist;
x) Optimieren des Netzwerks und Extrahieren der funktionalen Beziehung mit den UCLA-Schlaganfallrisiko-Scores.

2. Computerimplementiertes Verfahren zum Bestimmen eines Stress-d-Risiko-Index für das Risiko der Entwicklung von chronischem Stress und eines diabetesbedingten Schlaganfalls, aufweisend:
i) Verwenden des gemäß Anspruch 1 bestimmten Stress-Risiko-Index zum Ermitteln des Stress-d-Risiko-Index;
ii) Ausführen einer statistischen Analyse von Biomarkern und Risikofaktoren, wobei Variablen mit schiefen, nicht normalen Verteilungen logarithmisch transformiert wurden,
wobei die Biomarker und Risikofaktoren die Biomarker und Risikofaktoren des angepassten UCLA-Scores sind, mit den folgenden neun unabhängigen Markern: Alter, Geschlecht, systolischer Blutdruck, selbst angegebene Einnahme von blutdrucksenkenden Medikamenten, Diabetes, Rauchgewohnheiten, Durchblutungsstörungen, EKG-Vorhofflimmern und EKG-LVH, wobei die selbst angegebenen Variablen durch quantitative Marker ersetzt wurden, d.h. HbA1C ≥ 6,5% als ein Marker für Diabetes und Nikotinmetabolitcotinin ≥ 14 ng/ml als ein Marker für Rauchen, wobei zusätzlich das Leberenzym Gamma-Glutamyltransferase (GGT) als ein Marker für Alkoholmissbrauch verwendet wurde,
wobei die quantitativen Marker durch Messen in Nüchternblutproben bestimmt wurden: Serum-Cotinin-Werte, bestimmt durch ein homogenes Immunoassay, Gamma-Glutamyltransferase (GGT), gemessen in Serum, Lipide und hochsensitives c-reaktives Protein, gemessen in Vollblut-EDTA-Proben mit einem enzymatischen kolorimetrischen Assay, Gesamt-Insulin-ähnlicher Wachstumsfaktor-1, bestimmt in Serum unter Verwendung eines immunoradiometrischen Assays, und Vollblut-EDTA-glykiertes Hämoglobin (HbA1C), analysiert mit turbidimetrischen Hemmungs-Immunoassays;
iii) Bestimmen standardisierter Werte des angepassten UCLA-Scores durch Hauptkomponentenanalyse zu Beginn;
iv) Berechnen der ersten Hauptkomponenten-Scores als einen gewichteten Mittelwert standardisierter Variablen mit festgelegten Gewichten, die die folgenden sieben Komponentenbelastungen widerspiegeln: Cotinin, GGT, Diabetes definiert als HbA1C ≥ 6,5%, systolischer Blutdruck, Durchblutungsstörungen, Vorhofflimmern und EKG-LVH;
v) Bestimmen des Stress-d-Risiko-Index durch Multiplizieren der Komponenten-Scores mit zehn und Erhöhen um fünfzig, so dass der Mittelwert des Stress-d-Risiko-Index 50 beträgt und seine Standardabweichung zwischen 0 und minus 100 liegt;
vi) Bestimmen eines Schwellenwertes für den Stress-d-Risiko-Index durch Ausführen einer ROC-Analyse unter Verwendung des in Anspruch 1 iv) bestimmten Cut-off-Wertes, um zwischen positiven und negativen Daten und der Sensitivität, Spezifität und dem Prozentsatz korrekter Vorhersagen zu unterscheiden;
vii) Bezeichnen der dichotomen Variablen, die zwischen den Auskunftspersonen oberhalb des Schwellenwertes und denen unterhalb des Schwellenwertes unterscheidet, als Y, wobei der Stress-d-Risiko-Index AUC für eine positive Vorhersage 0,78 (95% CI 0,73, 0,83) betrug (81% Sensitivität, 59% Spezifität);
viii) Validieren des Stress-d-Risiko-Index durch Anwenden einer multivariaten linearen Regressionsanalyse in einem Modell unter Verwendung logarithmisch transformierter Prädiktoren in einem vollständigen Datensatz von N=349 unter Verwendung von Teilsätzen von 10 Trainingssätzen mit jeweils 60% Population und 10 Testsätzen mit den restlichen 40% Population;
ix) Anwenden einer logistischen linearen Regression in einem Modell unter Verwendung logarithmisch transformierter Prädiktoren in allen Daten wie in viii) unter Verwendung der Variablen Y wie in vii);
x) Validieren des logistischen linearen Regressionsmodells durch Wiederholen der Modellanpassung an 10 zufällig ausgewählte Proben und 10 Testsätze;
xi) Vorhersagen der Wahrscheinlichkeit des Risikos für chronischen Stress und diabetesbedingten Schlaganfall durch Ermitteln der Maximum-Likelihood-Schätzungen der Regressionskoeffizienten aller Regressionen;
xii) Verwenden des dichotomen Markers Y des Stress-d-Risiko-Index wie in vii) zum Unterscheiden zwischen den positiven und negativen Daten der Marker von xi);
xiii) Verwenden einer logistischen Regressionsanalyse, bei der Y als die abhängige Variable verwendet wird und V die ausgewählten kontinuierlichen Eingangs-Stress-Biomarker als Prädiktoren für positive Daten enthält, wobei der Stress-d-Risiko-Index AUC für eine positive Vorhersage 0,82 (95 % CI 0,75, 0,85) betrug (85% Sensitivität, 58% Spezifität);
xiv) Bestimmen eines optimalen Cut-off-Wertes für V unter Verwendung einer ROC-Analyse,
xv) Verwenden der Fläche unter der Kurve (AUC) in der ROC-Analyse für V unter Verwendung von Y sowie der Sensitivität, Spezifität und des Prozentsatzes korrekter Vorhersagen beim Cut-off-Wert als Diagnostik für die prädiktive Validität von V; und
xvi) Ausführen eines Hosmer-Lemeshow-Tests zum Testen der Anpassungsgüte der logistischen Regressionsmodelle zur Risikovorhersage bei allen Teilnehmern, Trainings- und Testdatensätzen.

## Revendications

1. Méthode mise en œuvre par ordinateur de détermination d'un indice STRESS^{risk} pour le risque de développer un accident vasculaire cérébral lié au stress chronique et à une cardiopathie ischémique, comprenant :
i) réaliser une régression linéaire multiple par étapes de biomarqueurs et de facteurs de risque, tous deux transformés pour être distribués normalement, d'un score UCLA adapté, ledit score UCLA étant un score composite de risque d'un accident vasculaire cérébral à 10 ans de l'Université de Californie, Los Angeles (UCLA), et incluant les variables suivantes :
antécédents médicaux individuels; i.e., maladie cardiovasculaire, maladie rénale, infarctus du myocarde, prise de médicaments contre l'hypertension et le diabète; facteurs démographiques et liés au mode de vie; i.e., âge, origine ethnique, sexe, diabète, tabagisme, consommation d'alcool et habitudes d'activité physique ; pression artérielle systolique et diastolique ; fibrinogène ; tour de taille ; déficits de perfusion, i.e., ischémie myocardique ; fibrillation atriale par électrocardiogramme ; et hypertrophie ventriculaire gauche par électrocardiogramme ;
dans laquelle les biomarqueurs et les facteurs de risque ont été déterminés en prélevant un échantillon biologique à jeun, i.e., sang, urine et salive, chez des individus ; en mesurant des concentrations des biomarqueurs suivants dans les échantillons biologiques prélevés chez les individus et en mesurant les niveaux des marqueurs cliniques suivants des individus ; les biomarqueurs étant des valeurs de cotinine sérique dérivés par un immunoessai homogène, de gamma-glutamyltransférase (γ-GT), de lipides et de protéine C-réactive (CRP) ultrasensible analysée par une méthode enzymatique, d'hémoglobine glyquée (HbA1c) sur sang total avec EDTA analysée par un immunoessai par inhibition turbidimétrique, des valeurs de fibrinogène citrate dérivés par une méthode immuno-turbimétrique à 540 nm par méthode de coagulation basée sur la viscosité, de cortisol salivaire analysé par un kit d'immunoessai par électrochimiluminescence, de cortisol sérique déterminé avec un kit d'immunoessai par électrochimiluminescence, de hormone adrénocorticotrope (ACTH) déterminée par un kit d'immunoessai par électrochimiluminescence, de troponine cardiaque à haute sensibilité déterminée par un kit d'immunoessai par électrochimiluminescence, dans laquelle l'urine acidifiée a été collectée pendant la nuit pour une analyse de la noradrénaline et de l'adrénaline urinaires à l'aide d'un kit ELISA FAST TRACK urinaire à 3 catécholamines, et dans laquelle la créatinine urinaire a été mesurée à l'aide de la méthode enzymatique, dans laquelle les marqueurs cliniques sont la pression artérielle systolique (SBP), la pression artérielle diastolique (DBP), toutes deux mesurées à l'aide d'un appareil ambulatoire combiné de mesure de la pression artérielle et d'électrocardiogramme, des événements d'ischémie myocardique silencieuse (SMI) ou des déficits de perfusion évalués par des enregistrements électrocardiographiques (ECG) à deux canaux sur 24 heures, électrocardiographie (ECG), fibrillation atriale identifiée par un ECG de repos à 12 dérivations, hypertrophie ventriculaire gauche déterminée par un ECG (ECG-LVH) à 12 dérivations, calibres des vaisseaux rétiniens mesurés sous forme d'images monochromes à l'aide d'un analyseur de vaisseaux rétiniens et d'un logiciel de cartographie des vaisseaux, pression intraoculaire (IOP) mesurée à l'aide d'un Tonomètre à Aplanation, et pression de perfusion oculaire diastolique (DOPP) calculée à partir de DBP moins IOP ;
ii) réaliser une analyse de la courbe ROC (caractéristique de fonctionnement du récepteur) pour évaluer la différence entre la distribution des biomarqueurs et la distribution des facteurs de risque à tous les seuils de classification ;
iii) déterminer l'indice STRESS^{risk} pour trois ou plus combinaisons possibles de biomarqueurs continus, sous forme d'aire sous la courbe (AUC), correspondant au maximum de l'analyse ROC, lors de la discrimination des résultats positifs et négatifs du biomarqueur composite dichotomique UCLA, noté Y, dans laquelle l'AUC de l'indice STRESS^{risk} était de 0,77 (95% CI 0,72, 0,82) pour une prédiction positive (sensibilité de 85 %, spécificité de 48 %) ;
iv) déterminer une valeur seuil par l'indice de Youden pour l'indice STRESS^{risk} maximisant des classifications correctes et/ou minimisant des classifications incorrectes et désignant la combinaison de biomarqueurs correspondant à ce seuil optimal comme biomarqueur V ;
v) valider un biomarqueur V en utilisant Y comme variable dépendante et V comme probabilité prédite de positifs, par régression logistique sur les biomarqueurs continus d'entrée sélectionnés et les facteurs de risque confondants ;
vi) discriminer l'AUC entre les positifs et les négatifs de Y en utilisant la probabilité prédite de positifs et en utilisant la sensibilité et la spécificité des prédictions correctes comme diagnostic de validité prédictive ;
vii) déterminer une valeur seuil optimale pour V à l'aide d'une analyse ROC ;
viii) utiliser un modèle de régression non linéaire incluant des réseaux de neurones comme comparaison avec le modèle de régression logistique ;
ix) déterminer le maximum de l'indice de Youden à l'aide des courbes ROC avec l'analyse de régression non linéaire étayant la relation fonctionnelle entre les modèles utilisant un perceptron multicouche à deux couches et entraîné avec une régularisation bayésienne, les couches cachées ayant des fonctions tansig et la couche de sortie étant linéaire avec dix répétitions bootstrap ;
x) optimiser le réseau et extraire la relation fonctionnelle avec les scores de risque d'accident vasculaire cérébral de l'UCLA.

2. Méthode mise en œuvre par ordinateur de détermination d'un indice STRESS^{d-RISK} pour le risque de développer un accident vasculaire cérébral lié au stress chronique et au diabète, comprenant :
i) utiliser l'indice STRESS^{risk} déterminé selon la revendication 1 pour établir l'indice STRESS^{d-RISK} ;
ii) réaliser une analyse statistique de biomarqueurs et de facteurs de risque, dans laquelle les variables présentant des distributions asymétriques non normales ont été transformées logarithmiquement ;
dans laquelle les biomarqueurs et les facteurs de risque sont les biomarqueurs et les facteurs de risque du score UCLA adapté, avec les neuf marqueurs indépendants suivants : âge, sexe, pression artérielle systolique, utilisation déclarée de médicaments antihypertenseurs, diabète, tabagisme, déficits de perfusion, fibrillation atriale par ECG et ECG-LVH, dans laquelle les variables déclarées ont été remplacées par des marqueurs quantitatifs, i.e. HbA1c ≥ 6,5 % comme marqueur du diabète et la cotinine métabolite de la nicotine ≥ 14 ng/ml comme marqueur du tabagisme, dans laquelle, en outre, l'enzyme hépatique gamma-glutamyltransférase (GGT) a été utilisée comme marqueur d'abus d'alcool ;
dans laquelle les marqueurs quantitatifs ont été déterminés en mesurant des échantillons de sang prélevés à jeun : des valeurs de cotinine sérique déterminés par un immunoessai homogène, de gamma-glutamyl transférase (GGT) mesurée dans du sérum, de lipides et de protéine c-réactive ultrasensible mesurés dans des échantillons de sang total avec EDTA avec un méthode enzymatique colorimétrique, de facteur de croissance 1 analogue à l'insuline total déterminé dans du sérum par une méthode immunoradiométrique, et d'hémoglobine glyquée (HbA1c) de sang total avec EDTA analysée par des immunoessais d'inhibition turbidimétrique ;
iii) déterminer des valeurs standardisées du score UCLA adapté par analyse en composantes principales à l'inclusion ;
iv) calculer les scores de la première composante principale comme moyenne pondérée des variables standardisées, les pondérations reflétant les sept contributions des composantes suivantes : cotinine, GGT, diabète défini par HbA1c ≥ 6,5 %, pression artérielle systolique, déficits de perfusion, fibrillation atriale et l'ECG-LVH ;
v) déterminer l'indice STRESS^{d-RISK} en multipliant les valeurs de scores des composantes par dix et en les augmentant de cinquante, de sorte que la moyenne de l'indice STRESS^{d-RISK} soit de 50 et son écart-type soit compris entre 0 et moins 100 ;
vi) déterminer une valeur seuil pour l'indice STRESS^{d-RISK} en effectuant une analyse ROC à l'aide de la valeur seuil déterminée dans la revendication 1 étape iv), afin de discriminer entre les données positives et négatives et la sensibilité, la spécificité et le pourcentage de prédictions correctes ;
vii) désigner la variable dichotomique qui discrimine entre les répondants au-dessus de la valeur seuil et ceux en dessous de la valeur seuil, comme Y, dans laquelle l'AUC de l'indice STRESS^{d-RISK} était de 0,78 (95 % CI 0,73, 0,83) pour une prédiction positive (sensibilité de 81 %, spécificité de 59 %) ;
viii) valider l'indice STRESS^{d-RISK} par application d'une analyse de régression linéaire multivariée dans un modèle utilisant des prédicteurs transformés logarithmiques sur un ensemble de données complet de N=349, en utilisant des sous-ensembles de 10 ensembles d'entraînement avec chacun 60 % de la population et 10 ensembles de test avec les 40 % restants de la population ;
ix) appliquer une régression logistique linéaire dans un modèle utilisant des prédicteurs transformés logarithmiques sur toutes les données comme en viii) en utilisant la variable Y comme en vii) ;
x) valider le modèle de régression logistique linéaire en répétant l'ajustement du modèle sur 10 échantillons sélectionnés aléatoirement et 10 ensembles de test ;
xi) prédire la probabilité de risque d'accident vasculaire cérébral lié au stress chronique et au diabète en obtenant les estimations de vraisemblance maximale des coefficients de régression de toutes les régressions ;
xii) utiliser le marqueur dichotomique Y de l'indice STRESS^{d-RISK} comme dans vii) afin de discriminer entre les résultat positifs et négatifs des marqueurs de l'étape xi);
xiii) utiliser une analyse de régression logistique dans laquelle Y est utilisé comme variable dépendante et V contient les biomarqueurs de stress continus sélectionnés comme prédicteurs de résultats positifs, dans laquelle l'AUC de l'indice STRESS^{d}-^{risk} était de 0,82 (95 % CI 0,75, 0,85) pour une prédiction positive (sensibilité de 85 %, spécificité de 58 %) ;
xiv) déterminer une valeur seuil optimale pour V à l'aide d'une analyse ROC ;
xv) utiliser l'aire sous la courbe (AUC) de l'analyse ROC sur V, en utilisant Y, et la sensibilité, la spécificité et le pourcentage de prédictions correctes à la valeur seuil comme diagnostics de la validité prédictive de V ; et
xvi) réaliser un test de Hosmer-Lemeshow pour tester la qualité d'ajustement des modèles de prédiction du risque de régression logistique dans tous les participants, ensembles d'entraînement et de test.
